# EUROPEAN PATENT SPECIFICATION

(11) **EP 1 786 397 B1**
(45) Date of publication and mention of the grant of the patent: **18.05.2011**
(21) Application number: 05776796.4
(22) Date of filing: 02.08.2005
(51) Int. Cl.: A61K 9/06, A61K 9/00, A61K 31/415, A61K 47/38, A61K 47/40, A61K 47/42, A61K 9/12, A61K 9/107

(54) **PHENYTOIN FORMULATIONS, AND USES THEREOF IN WOUND HEALING**
PHENYTOINFORMULIERUNGEN UND IHRE VERWENDUNGEN IN DER WUNDHEILUNG
FORMULATIONS DE PHENYTOINE, ET UTILISATIONS ASSOCIEES POUR GUERIR DES BLESSURES

(30) Priority: 03.08.2004 US 598479 P
(43) Date of publication of application: 23.05.2007
(73) Proprietor: Royal College of Surgeons in Ireland, Dublin 2 (IE)
(72) Inventor: KELLY, John, Drumcondra, Dublin 9 (IE); KENNEDY, Cormac, Ballysheedy, Co. Limerick (IE); MEANEY, Clare, Dublin 4 (IE)
(74) Representative: Purdy, Hugh Barry
(86) International application number: PCT/EP2005/008344
(87) International publication number: WO 2006/013084

(56) References cited:
- EP-A- 0 515 758
- GB-A- 2 327 344
- US-A1- 2003 186 955
- US-B1- 6 730 323

## Description

### Technical Field

This invention relates to formulations of phenytoin suitable for topical application to a wound. In particular the invention relates to gel-based formulations of phenytoin. The invention also relates to gel-based formulations of phenytoin for use in methods for the topical treatment of wounds.

### Background Art

Phenytoin (diphenylhydantoin, 5,5-diphenylimidazolidine-2, 4-dione) has well-established clinical use as an anticonvulsant and is used as an anti epileptic agent. It also has cardiac antiarrhythmic properties. Phenytoin can be administered orally or (as the sodium salt) by injection.

Despite the usefulness of phenytoin for treating seizures, it has a number of side effects, which are well described in the literature (see for example Goodman & Gilman's The Pharmacological Basis of Therapeutics, McGraw Hill). These side effects include gastrointestinal disturbances, nervous system toxicity, various central nervous system effects, various endocrine effects, hirsutism, cardiac arrhythmias, liver damage, hypersensitivity reactions and interactions with other medications.

One frequently observed side effect is tenderness and enlargement of the tissues of the gums (gingival hyperplasia). Phenytoin is the most common cause of drug-induced gingival enlargement. However it is also associated with use of cyclosporin and calcium channel blockers (Desai P & Silver JG, J Can Dent Assoc. 69(4): 263-8, 1998).

At least 20% and perhaps up to about 50% of patients treated with phenytoin develop gingival enlargement as a side effect. The incidence may increase as the plasma concentration of phenytoin increases (Perlik F et al. Ther Drug Monit. 17(5) 445-8 1995). This stimulatory effect on tissue growth has prompted the evaluation of phenytoin in wound healing.

The literature contains result of clinical investigations and case reports of treatments in which the application of phenytoin had beneficial properties in wound healing in various types of wounds. These include experimental models of incised wounds (DaCosta ML, Surgery 123(3) 287-93 1998) and fractures (Frymoyer JW, J Trauma 16(5) 368-70, 1976) and clinical studies in traumatic wounds (Modaghegh S, et al. Int J Dermatol. 28(5) 347-50 1989), ulcers (Anstead GM, et al. Ann Pharmacother. 30(7-8.) 768-75 1996; Bansal NK, Int J Dermatol. 32(3): 210-3,1993) and skin grafts (Yadav JK, Burns, 19(4): 306-10, 1993).

The mechanism of action of phenytoin in wound healing is unknown but is likely to be related to the otherwise adverse effect of gingival tissue enlargement rather than to its anticonvulsant properties. A number of studies have shown that phenytoin increases the production of various cytokines and growth factors, including interleukin -1b (Modeer T, et al. Life Sci. 44(1) 35-401 1989), keratinocyte growth factor (Das SJ and Olsen I, Biochem Biophys Res Commun. 13 282(4) 875-81 2001), platelet-derived growth factor (Iacopino AM, et al. J Periodontol. 68(1) 73-83 1997) and basic fibroblast growth factor (Sasaki T and Maita E, J Clin Periodontol. 25(1) 42- 7 1998). Phenytoin altered the expression of a number of genes in dermal fibroblasts (Swamy SMK, et al. Biochem Biophys Res Commun. 314 661-666 2004).

The above investigations employed phenytoin or a salt contained in or added to a biological medium in the case of in vitro studies, applied topically as a solution, suspension or powder for in vivo or clinical studies, or in some cases administered by injection, for example in studies with bone fractures. The liquid or powder applications are mostly derived from the commercially available injections or capsules. They do not teach how phenytoin may be used in a suitable pharmaceutical composition that can be conveniently and accurately used in the treatment of wounds in patients who would benefit from such treatment. Lasker (US Patent No. 5,571,521) describes the use of a silver ammonium phenytoin complex, together with phenytoin, in the treatment of wounds and particularly as biocides for the treatment of infections. However no description is given of the pharmaceutical compositions necessary to achieve these ends.

Phenytoin is only slightly soluble in water. Phenytoin sodium salt is soluble in water but only remains in solution in significant amounts at alkaline pH, typically at pH values of approximately 12. Exposure of wound tissue to this pH is not desirable. At lower values of pH there is an increased proportion of undissolved phenytoin.

Given the acknowledged efficacy of phenytoin in promoting the healing of wounds, there is a clear need in the art for methods whereby this property may be provided in a convenient, efficacious, stable and reproducible manner for use by patients and their carers. Preferably there should be means to ensure a suitable residence time and a method of ensuring uptake of the agent by the cells and tissues of the wound.

### Statements of Invention

According to the invention, there is provided a formulation of phenytoin according to claim 1.

In this specification, the term "wound", unless otherwise indicated, is intended to mean both chronic wounds such as those associated with long-term illness such as diabetes and those associated with immobility such as decubitus ulcers, and acute wounds such as those of a traumatic origin.

Typically, the gel is formed by a gelling agent selected from the group comprising: alginic acid; alginate derivatives; chitosan; chitosan derivatives; and a carbomer.

Generally, the gel-forming gelling agent is present in the formulation in an amount of from 0.5% to 10.0% (w/w), suitably from 0.5% to 5%, preferably from 0.5% to 3%, more preferably from 0.5% to 2%, and ideally at about 1% (w/w).

In one embodiment of the invention, the gel includes a complexing agent which functions to physically entrap the phenytoin in the reservoir. Suitable stabilising agent may be selected from the group comprising: cyclodextrins; buffer salts; amino acids; small peptides; polyarginines; polyglycines; polylysines; and glutamic acid. A particularly suitable complexing agent is hydroxypropyl-β-cyclodextrin. Typically, the formulation will include a complexing agent in an amount of from 1% to 50% (w/w), preferably from 20% to 40% (w/w), and more preferably from 25% to 35% (w/w). In one preferred embodiment of the invention, the formulation will include a complexing agent along with one or more of gelling agents. In this regard, formulations that include a carbomer and a cyclodextrin have been found to be particularly advantageous.

In one embodiment, the phenytoin is present in the formulation at an amount of from 0.5% to 10.0%, preferably from 1.0% to 7.0%, and more preferably from 2.0% to 6.0% (w/w). Ideally, the phenytoin is present at an amount of from 3.0% to 5.0% (w/w). Suitably, the phenytoin comprises phenytoin acid, a phenytoin salt, a derivative of phenytoin, or a mixture thereof. In one embodiment, the derivative of phenytoin includes pro-drugs such as that sold under the Trade Name FOSPHENYTOIN. Ideally, the phenytoin salt consists of sodium phenytoin.

In one embodiment, the formulation of the invention includes one or more therapeutic molecules for the treatment of wounds, such as, for example, anti-infective agents selected from the group comprising antibiotics, antifungal agents, and zinc salts.

In one particularly suitable embodiment of the invention, the formulation comprises:
- from 0.5% to 10.0% phenytoin salt:
- from 0.5% to 5.0% gelling agent;
- an aqueous base; and optionally
- an amount of alkali or acid or buffer salts sufficient to adjust the pH of the formulation to between 7 and 10, in which the gelling agent comprises a polymer that is capable of forming an ion-pair with the phenytoin. Examples of such gelling agents include carbomers (i.e. CARBOPOL) and alginates.

Suitably, the formulation additionally includes a complexing agent for the phenytoin. A preferred complexing agent is a cyclodextrin.

Ideally, the gelling agent comprises from 0.5% to 1.5% of the formulation (w/w). Generally, the cyclodextrin may be present at from 20% to 40% (w/w).

The invention also relates to a.solid support forming part of a bandage or a dressing for wounds, wherein the solid support carries a formulation according to the invention. Typically, the solid support comprises a mesh formed of woven or non-woven material. The invention also relates to a bandage or dressing for wounds comprising a solid support according to the invention.

The invention also relates to a formulation of the invention for use as a medicament.

The invention also relates to a formulation of the invention for use in the topical treatment of wounds.

A formulation according to the invention for use in the topical treatment of wounds in diabetic patients.

The present invention takes the form of formulations of phenytoin in which there is an equilibrium among dissolved and suspended phenytoin, phenytoin sodium and free phenytoin. The relative proportions of all of these depend upon the pH, the original concentrations of phenytoin and phenytoin sodium and any solubilising excipients in the formulation. These multi component systems contain a slowly releasing reservoir of phenytoin in equilibrium with dissolved phenytoin in the formulation and further in equilibrium with phenytoin in the physiological environment of the wound, including the physiological fluid surrounding the cells and tissue of the wound, the cell membrane and the intracellular environment. The present invention encompasses topical formulations of phenytoin having pH values in the range 7-10, which contain phenytoin free substance or the sodium salt or a combination of these, such that there is an equilibrium between dissolved and suspended phenytoin.

The invention is a topical, extended release formulation of phenytoin in which there is an equilibrium among a phenytoin reservoir and free phenytoin in the formulation and in the physiological environment of the wound. The composition of the reservoir influences the amount of phenytoin available to the wound and the time course of the exposure of the wound to the therapeutic effect of phenytoin.

The components of the formulation are as follows
1. A reservoir of phenytoin. This may take the form of a suspension of phenytoin or a salt of phenytoin and may be a reservoir in which phenytoin or a salt is included in a matrix in a complexed or intercalated form such as that formed by mixing with a cyclodextrin.
2. Free phenytoin or a salt of phenytoin dissolved in the formulation. This is in equilibrium with the phenytoin contained in the reservoir. The relative amounts of phenytoin in each component will be influenced by the composition of the formulation, including the pH and the presence of excipients that influence the solubility of phenytoin.

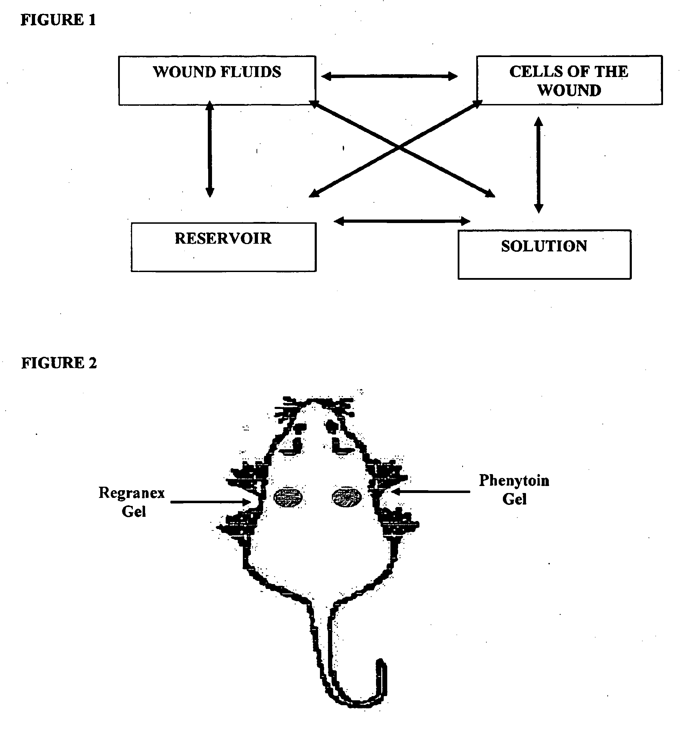

Upon application to the wound a further set of equilibrium conditions are established among phenytoin or its salt in the reservoir, dissolved in the formulation, suspended or dissolved in the physiological fluid surrounding the cells and tissues of the wound and phenytoin in the cell membranes and the intracellular environment of the wound. A diagram illustrating the set of equilibrium conditions is shown in Figure 1.

The operation of the invention following application to the wound is illustrated in the above figure. Phenytoin is present in all of the compartments and the equilibrium conditions can be varied by adjusting the various constituents of the formulation. For example the ultimate duration of action can be made dependent upon the amount of phenytoin in the reservoir. The amount of phenytoin made available to the cells and tissue of the wound can be made dependent upon the pH of the formulation or the nature and amount of excipients.

While the application of this invention to phenytoin and phenytoin sodium and to suspensions of phenytoin and cyclodextrin complexes is described above, its applicability to other salts and complexes of phenytoin and its analogues may readily be appreciated and the incorporation of additional therapeutic molecules for the treatment of wounds is envisaged. Notable among these is the use of antimicrobial substances, for example anti-infective agents such as antibiotics, antifungals and zinc salts.

Gels provide a particularly useful form of the invention. They are semisolid and liquid rich and form a suitable compatible formulation for application to wounds. They can be prepared with a range of viscosities. Their formulations may contain solvents, emulsifiers, moisturizers, emollients, preservatives and other active ingredients that increase or enhance the efficacy of the final product. The presence of solvents can contribute to the modulation of release rates. The invention contemplates topical formulations designed for controlled-release of phenytoin to a wound surface.

As mentioned above, gels are a particularly useful form of the invention. It can be readily understood that the gel may be presented in various forms and viscosities that range from the essentially liquid to relatively solid. It may be squeezed from a deformable multiple application container, from suitable unit dose containers or applied as sheets or be prepared on solid matrices, such as dressings. The above are examples and are not intended to form an exhaustive list but serve to illustrate the methods.

Surprisingly the Applicant has discovered that the use of a Carbomer has a counter-ion effect that results in a stable, smooth dispersion of phenytoin at pH values close to physiological, in contrast to simple aqueous preparations. Therefore the use of a Carbomer as a gelling agent is particularly advantageous and these preparations constitute a preferential form of the present invention.

Surprisingly also we have discovered that the invention displays particular utility in the treatment of wounds in diabetic conditions and the use of the invention in these wounds is a preferential but not exclusive indication.

By altering the various components of the formulation, products of differing durations of action can be produced and extended release products offering convenience to patients and their carers are envisaged. Various frequencies of administration can be envisaged from say once daily to once weekly or indeed as a single administration. These are examples and do not seek to confine the frequency or total number of administrations.

### Detailed Description of the Invention

The invention will be more clearly understood from the following description of some embodiments thereof, given by way of example only, with reference to the accompanying figures and graphs.

### EXAMPLE 1 Extended-release phenytoin gel.

Carbomer 974 PNF 1g is added to approximately 80g deionised water. Following complete addition of Carbomer, the preparation is mixed for 30 minutes. Phenytoin sodium 5g is gradually added while mixing. The gel thickens. The gel is made up to 100g with deionised water, with mixing. The pH is adjusted to 7.4 with sodium hydroxide.

### EXAMPLE 2. Extended-release phenytoin gel.

Hydroxypropyl-β-Cyclodextrin 30g is dissolved in approximately 60g deionised water with stirring. Phenytoin sodium 1g is added slowly to form a suspension. Carbomer 974 PNF 1g is gradually added with mixing at 1500rpm. The gel is made up to 100g with I deionised water, with mixing. The pH is adjusted to 7.4 with sodium hydroxide.

### EXAMPLE 3. Efficacy model.

A rat dorsal wound model as described by DaCosta et al. (Surgery 123(3) 287-93 1998) was employed. Wounds were treated by insertion of aliquots (0.2g) of either the phenytoin-containing gel described in Example 2 above, or a gel manufactured without phenytoin. Ten days afterwards, the integrity of the wounds were examined and compared. The beneficial effects of the invention were manifested by an increase in wound tensile strength of approximately 30%, when compared with the inactive treatment, accompanied by increased amounts of hydroxyproline, which is a marker of collagen deposition.

### EXAMPLE 4. Efficacy model - diabetes.

A full-thickness excisional wound were studied in rats rendered diabetic by the administration of streptozotocin. Wounds were treated by application of aliquots of either the phenytoin-containing gel described in Example 2 above, or a gel manufactured without phenytoin. The improvement in wound healing in the diabetic animal (demonstrated by a decrease in wound area) was at least 50% greater than placebo-treated animals at day 9 and day 12.

### EXAMPLE 5 Comparison with commercially available product

Male Sprague-Dawley rats were rendered diabetic by a single intraperitoneal injection of streptozotocin (STZ). Serum glucose was measured from a sample of venous blood taken from the tail vein of each diabetic rat one week after STZ administration using a glucometer and dextroslide. In this excisonal wound model, two circular wounds with a diameter of 20mm were created at equal distance from the midpoint and symmetrically on the dorsum of the rat below the inferior edge of the scapula, as shown in Figure 2. Treatments were topically applied once daily to the wounds. The area of the wound was traced onto a transparent sheet every third day and the tracing scanned. Wound area was calculated with a non-rectangular area analysis. The serum concentration of phenytoin was measured by sampling at the end of the experimental period. Blood was draw by intra-cardiac puncture biochemically analysed for phenytoin. All animals were housed in a licensed biomedical research facility under normal laboratory conditions and the study was conducted with the approval of our institutional ethics committee.

Conventional diabetic and normal rat models were used for an excisonal wound healing study. Two circular wounds are created on each animal, one was treated with a commercially available wound healing product, REGRANEX, and the other with 5% phenytoin sodium gel of Example 1. Regranex is a preparation containing becaplermin or human platelet derived growth factor. It was found that there was no significant difference in the time to healing for diabetic animals (p=0.29).

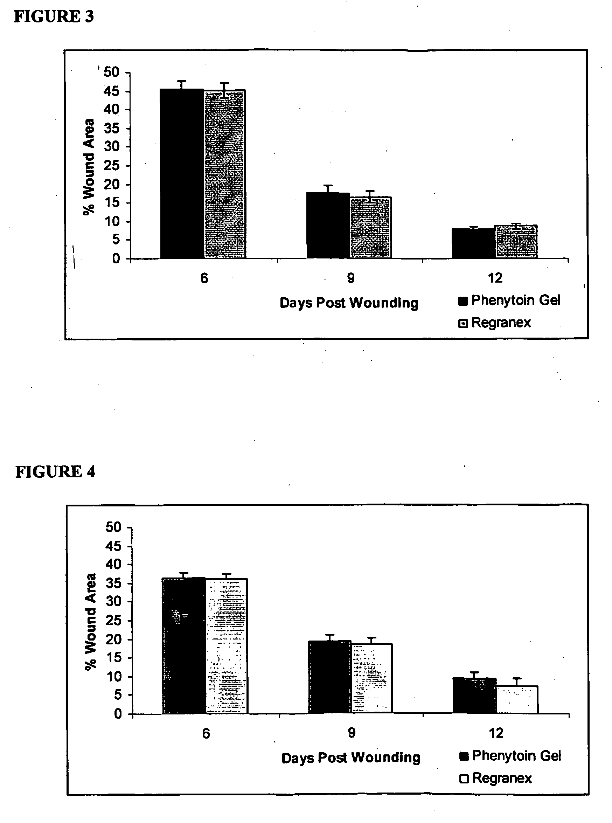

The percentage of wound area remaining for those animals treated daily with phenytoin gel equated with that for those treated with Regranex (Figure 3). Also, there was no significant difference between the wound areas for the normal rat model (p>0.05 for all days, Figure 4).

### EXAMPLE 6 Controlled release properties

The release of phenytoin sodium from the gels into a basic medium was investigated over a 24 hour period.

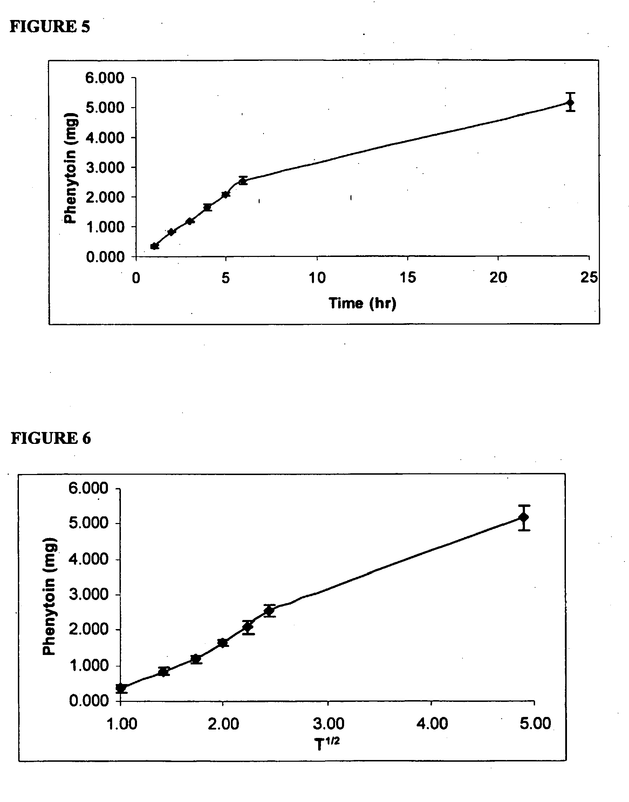

Figure 5 illustrates the release profile of phenytoin sodium from a 5% phenytoin sodium 1% carbomer gel over a 24 hour period.

Figure 6 illustrates the release profile of phenytoin sodium from a 5% phenytoin sodium 1% carbomer gel over a 24 hour period plotting drug released versus the root of time.

9% of the drug present in the gel applied was released over the 24 hour period.

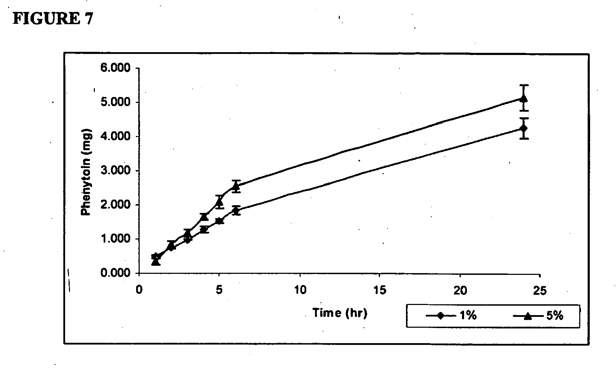

Figure 7 provides a comparison of the release profile of phenytoin sodium from 5% phenytoin sodium and 1% phenytoin sodium gels over a 24 hour period.

The rate of release of the drug seems to be independent of the drug concentration after the first six hours, possibly showing the rate of transfer of the drug from the suspended reservoir to the soluble phase becomes constant.

## Claims

1. A formulation of phenytoin suitable for topical application to a wound comprising a reservoir of phenytoin entrapped within a gel, and an amount of phenytoin dissolved in an aqueous component of the gel, wherein the dissolved phenytoin is in chemical equilibrium with the phenytoin entrapped within the gel, in which the gel is formed by a gelling agent suitable for forming an ion pair with the phenytoin, wherein the formation of ion-pairs between the gelling agent and the phenytoin molecules increases the stability of the reservoir of phenytoin, wherein the formulation has a pH of from 7 to 10, and wherein the gel comprises an acidic polymer.

2. A formulation as claimed in Claim 1 in which the gel is formed by a gelling agent selected from the group comprising: alginic acid; alginate derivatives; chitosan; chitosan derivatives; and a carbomer.

3. A formulation as claimed in Claim 2 in which the carbomer gelling agent comprises CARBOPOL.

4. A formulation as claimed in any of Claims 1 to 3 in which the gel includes a complexing agent which physically entraps the phenytoin in the reservoir.

5. A formulation as claimed in Claim 4 in which the complexing agent is selected from the group comprising: cyclodextrins; buffer salts; amino acids; small peptides; polyarginines; polyglycines; polylysines; and glutamic acid.

6. A formulation as claimed in any preceding Claim in which the gelling agent is present in an amount of from 0.5% to 5.0.% (w/w).

7. A formulation as claimed in any preceding Claim in which the phenytoin is present in the formulation at an amount of from 0.5% to 10.0% (w/w).

8. A formulation as claimed in any preceding Claim in which the phenytoin is a phenytoin salt.

9. A formulation as claimed in Claim 8 in which the phenytoin salt consists of sodium phenytoin.

10. A formulation as claimed in any preceding Claim further including one or more therapeutic molecules for the treatment of wounds.

11. A formulation as claimed in Claim 10 in which the therapeutic molecules are anti-infective agents selected from the group comprising antibiotics, antifungal agents, and zinc salts.

12. A formulation as claimed in Claim 1, comprising:
- from 0.5% to 10.0% phenytoin salt;
- from 0.5% to 5.0% gelling agent;
- an aqueous base; and optionally
- an amount of alkali or acid or buffer salts sufficient to adjust the pH of the formulation to between 7 and 10,
wherein the gelling agent comprises an acidic gel-forming polymer capable of forming an ion pair with the phenytoin.

13. A formulation as claimed in Claim 12 in which the gelling agent is a carbomer.

14. A solid support forming part of a bandage or a dressing for wounds, wherein the solid support carries a formulation according to any of Claims 1 to 13.

15. A solid support as claimed in Claim 14 and comprising a mesh formed of woven or non-woven material.

16. A bandage or dressing for wounds comprising a solid support as claimed in Claim 14 or 15.

17. A formulation as claimed in any of Claims 1 to 13 for use as a medicament.

18. A formulation as claimed in any of Claim 1 to 13 for use in the topical treatment of wounds.

19. A formulation as claimed in any of Claims 1 to 13 for use in the topical treatment of wounds in diabetic patients.

## Patentansprüche

1. Formulierung aus Phenytoin, die für eine topische Anwendung auf eine Wunde geeignet ist und ein in einem Gel eingeschlossenes Phenytoin-Reservoir und eine in einer wässrigen Komponente des Gels aufgelöste Menge an Phenytoin umfasst, wobei das gelöste Phenytoin in einem chemischen Gleichgewicht mit dem in dem Gel eingeschlossenen Phenytoin ist, wobei das Gel durch ein Geliermittel gebildet wird, das zur Bildung eines Ionenpaares mit dem Phenytoin geeignet ist, wobei die Bildung von Ionenpaaren zwischen dem Geliermittel und den Phenytoinmolekülen die Stabilität des Phenytoin-Reservoirs erhöht, wobei die Formulierung einen pH-Wert von 7 bis 10 hat und wobei das Gel ein saures Polymer umfasst.

2. Formulierung nach Anspruch 1, wobei das Gel durch ein Geliermittel gebildet wird, das ausgewählt ist aus der Gruppe umfassend: Alginsäure; Alginatderivaten; Chitosan; Chitosanderivaten; und einem Carbomer.

3. Formulierung nach Anspruch 2, wobei das Carbomer-Geliermittel CARBOPOL umfasst.

4. Formulierung nach einem der Ansprüche 1 bis 3, wobei das Gel einen Komplexbildner beinhaltet, der das Phenytoin in dem Reservoir physisch einschließt.

5. Formulierung nach Anspruch 4, wobei der Komplexbildner augewählt ist aus der Gruppe umfassend: Cyclodextrinen; Puffersalzen; Aminosäuren; kleinen Peptiden; Polyargininen; Polyglycinen; Polylysinen; und Glutaminsäure.

6. Formulierung nach einem der vorherigen Ansprüche, wobei das Geliermittel in einer Menge von 0,5 % bis 5,0 % (w/w) vorliegt.

7. Formulierung nach einem der vorherigen Ansprüche, wobei das Phenytoin in der Formulierung in einer Menge von 0,5 % bis 10,0 % (w/w) vorliegt.

8. Formulierung nach einem der vorherigen Ansprüche, wobei das Phenytoin ein Phenytoinsalz ist.

9. Formulierung nach Anspruch 8, wobei das Phenytoinsalz aus Natrium-Phenytoin besteht.

10. Formulierung nach einem der vorherigen Ansprüche, die ferner ein oder mehrere therapeutische Moleküle zur Behandlung von Wunden beinhaltet.

11. Formulierung nach Anspruch 10, wobei die therapeutischen Moleküle Antiinfektiosa sind, die ausgewählt sind aus der Gruppe umfassend Antibiotika, Antipilzmitteln und Zinksalzen.

12. Formulierung nach Anspruch 1, die Folgendes umfasst:
- 0,5 % bis 10,0 % Phenytoinsalz;
- 0,5 % bis 5,0 % Geliermittel;
- eine wässrige Base; und optional
- eine Menge an Alkali oder Säure oder Puffersalzen, die ausreicht, um den pH-Wert der Formulierung zwischen 7 und 10 einzustellen,
wobei das Geliermittel ein saures gelbildendes Polymer umfasst, das mit dem Phenytoin ein Ionenpaar bilden kann.

13. Formulierung nach Anspruch 12, wobei das Geliermittel ein Carbomer ist.

14. Fester Träger, der Teil einer Bandage oder eines Verbands für Wunden bildet, wobei der feste Träger eine Formulierung nach einem der Ansprüche 1 bis 13 trägt.

15. Fester Träger nach Anspruch 14, der ein Netz aus gewebtem oder ungewebtem Material umfasst.

16. Bandage oder Verband für Wunden, die/der einen festen Träger nach Anspruch 14 oder 15 umfasst.

17. Formulierung nach einem der Ansprüche 1 bis 13 für den Gebrauch als Medikament.

18. Formulierung nach einem der Ansprüche 1 bis 13 für den Gebrauch bei der topischen Behandlung von Wunden.

19. Formulierung nach einem der Ansprüche 1 bis 13 für den Gebrauch bei der topischen Behandlung von Wunden bei Diabetikern.

## Revendications

1. Formulation de phénytoïne convenant pour une application topique sur une plaie, comprenant un réservoir de phénytoïne piégé à l'intérieur d'un gel et une quantité de phénytoïne dissoute dans un composant aqueux du gel, la phénytoïne dissoute étant en équilibre chimique avec la phénytoïne piégée à l'intérieur du gel, le gel étant formé par un agent gélifiant convenable pour la formation d'une paire d'ions avec la phénytoïne, la formation de paires d'ions entre l'agent gélifiant et les molécules de phénytoïne augmentant la stabilité du réservoir de phénytoïne, la formulation présentant un pH allant de 7 à 10, et le gel comprenant un polymère acide.

2. Formulation selon la revendication 1, dans laquelle le gel est formé d'un agent gélifant choisi dans le groupe comprenant : l'acide alginique; les dérivés d'alginate; le chitosane ; les dérivés du chitosane ; et un carbomère.

3. Formulation selon la revendication 2, dans laquelle l'agent gélifiant carbomère comprend le CARBOPOL.

4. Formulation selon l'une quelconque des revendications 1 à 3, dans laquelle le gel inclut un agent complexant qui piège physiquement la phénytoïne dans le réservoir.

5. Formulation selon la revendication 4, dans laquelle l'agent complexant est choisi dans le groupe comprenant : les cyclodextrines ; les sels tampons ; les acides aminés ; les peptides de petite taille ; les polyarginines ; les polyglycines ; les polylysines ; et l'acide glutamique.

6. Formulation selon l'une quelconque des revendications précédentes, dans laquelle l'agent gélifiant est présent en une quantité allant de 0,5 % à 5,0 % (p/p).

7. Formulation selon l'une quelconque des revendications précédentes, dans laquelle la phénytoïne est présente dans la formulation en une quantité allant de 0,5 % à 10,0 % (p/p).

8. Formulation selon l'une quelconque des revendications précédentes, dans laquelle la phénytoïne est un sel de phénytoïne.

9. Formulation selon la revendication 8, dans laquelle le sel de phénytoïne est constitué de phénytoine sodique.

10. Formulation selon l'une quelconque des revendications précédentes, comprenant en outre une ou plusieurs molécules thérapeutiques pour le traitement de plaies.

11. Formulation selon la revendication 10, dans laquelle les molécules thérapeutiques sont des agents anti-infectieux choisis dans le groupe comprenant les antibiotiques, les agents antifongiques et les sels de zinc.

12. Formulation selon la revendication 1, comprenant :
- de 0,5 % à 10,0 % de sel de phénytoïne ;
- de 0,5 % à 5,0 % d'agent gélifiant ;
- une base aqueuse ; et éventuellement
- une quantité suffisante de sels alcalins ou acides ou tampons pour ajuster le pH de la formulation entre 7 et 10,
l'agent gélifiant comprenant un polymère acide formant un gel capable de former une paire d'ions avec la phénytoïne.

13. Formulation selon la revendication 12, dans laquelle l'agent gélifiant est un carbomère.

14. Support solide formant une partie d'un bandage ou d'un pansement pour plaies, le support solide portant une formulation selon l'une quelconque des revendications 1 à 13,

15. Support solide selon la revendication 14, comprenant un maillage formé d'un matériau tissé ou non tissé.

16. Bandage ou pansement pour plaies comprenant un support solide selon la revendication 14 ou 15.

17. Formulation selon l'une quelconque des revendications 1 à 13, destinée à être utilisée comme médicament.

18. Formulation selon l'une quelconque des revendications 1 à 13, destinée à être utilisée pour le traitement topique de plaies.

19. Formulation selon l'une quelconque des revendications 1 à 13, destinée à être utilisée pour le traitement topique de plaies chez les patients diabétiques.
